# EUROPEAN PATENT APPLICATION

(11) **EP 3 628 214 A1**
(43) Date of publication of application: **01.04.2020**
(21) Application number: 18197708.3
(22) Date of filing: 28.09.2018
(51) Int. Cl.: A61B 5/00, A61B 6/00, A61B 5/05, A61B 5/053, A61B 6/03

(54) **RECONSTRUCTION OF A LOW-ENERGY IMAGE BASED ON A COMPUTER TOMOGRAPHY IMAGE**

(71) Applicant: Siemens Healthcare GmbH, 91052 Erlangen (DE)
(72) Inventor: Schmidt, Sebastian, 91085 Weisendorf (DE); Hofmann, Bernd, 91058 Erlangen (DE); Müller, Kerstin, 90443 Nürnberg (DE); Della Monta, Christophe, 91336 Heroldsbach (DE); Geissenberger, Caroline, 91054 Erlangen (DE); Schmolke, Susanne, 91052 Erlangen (DE); Zhao, Yufan, 91052 Erlangen (DE)

(57) **Abstract**

A method for reconstructing a low-energy image comprises:
- Receiving the low-energy imaging data of an area of interest that is based on an interaction of the area of interest with low-energy photons,
- Receiving a computed tomography image of the area of interest,
- Generating, based on the computed tomography image, a tissue characteristics map of the area of interest regarding the interaction with the low-energy photons, and
- Reconstructing, based on the low-energy imaging data and the tissue characteristics map, the low-energy image.

## Description

In one aspect the invention relates to a method for reconstructing a low-energy image. In one further aspect the invention relates to an imaging data processing unit. In one further aspect the invention relates to a medical imaging system.

Many imaging modalities exist which are in principle useful for the diagnosis of stroke or other neurological diseases. However, they all have restrictions on their usability in practice. Magnetic resonance imaging (MRI) offers most information, but has contraindications, availability in an emergency situation is not always given and temporal resolution is very low. Computed tomography (CT) is fast and has good availability, but limited soft-tissue contrast and functional information. Functional information may include, for example, cortical activity derived from reduction of oxygen saturation and/or localization of seizure foci by changes in evoked potentials.

New concepts like near-infrared imaging, microwave tomography or electric impedance tomography are also known and offer sometimes also functional information, but have a very limited spatial resolution, which makes them not useful for localization of lesions or diagnosis of smaller lesions. Reason for this is that electromagnetic waves at low energies (e.g. infrared light, microwaves etc. with photon energies <10 eV) are mainly influenced by scattering (Rayleigh effect). As the direction of the photon is changed by every scattering event, it is very challenging to localize the scattering event from the received photon.

CT and X-ray, however, use higher energy photons (30-140 keV), which are mainly influenced by absorption (Compton Effect, photo effect), which gives them a more ballistic behavior and makes it easier to localize the absorption events in space. However, because of the other kinds of interaction with tissue, you get a different contrast, which is mainly based on X-ray absorption, which has limited correlation with function.

US 2015/0265228 A1 discloses a method for registering a near-infrared spectroscopy map and an anatomical image data record.

The underlying technical problem of the invention is to facilitate an improved reconstruction of an image based on low-energy photon imaging data. This problem is solved by the subject matter of each of the independent claims. The dependent claims are related to further aspects of the invention.

In one aspect the invention relates to a method for reconstructing a low-energy image, comprising:
- Receiving the low-energy imaging data of an area of interest that is based on an interaction of the area of interest with low-energy photons,
- Receiving a computed tomography image of the area of interest,
- Generating, based on the computed tomography image, a tissue characteristics map of the area of interest regarding the interaction with the low-energy photons, and
- Reconstructing, based on the low-energy imaging data and the tissue characteristics map, the low-energy image.

One major challenge of reconstructing an image based on the low-energy photon imaging data is, that the tissue characteristics in respect of an interaction with low-energy photons of the different tissue types along a low-energy photon path are typically extremely different, but not a priori known. This can be improved by using the computed tomography image. In particular, tissue characteristics map regarding, for example, the scattering and/or the absorption characteristics of the tissue of the area of interest, can be determined based on the computed tomography image. The tissue characteristics map can comprise, for each voxel of the computed tomography image, at least one corresponding tissue characteristics value. Therefore, information from the computed tomography image can be used in the reconstruction of the image based on the low-energy photon imaging data.

In a further aspect the reconstructing the image based on the low-energy imaging data and the tissue characteristics map comprises:
- Generating, based on the tissue characteristics map, an initial estimate image of the area of interest,
- Calculating virtual low-energy imaging data based on the initial estimate image,
- Comparing the calculated virtual low-energy imaging data to the received low-energy imaging data, thereby obtaining a comparison result,
- Generating, based on the comparison result, a refined estimate image of the area of interest.

Based on the refined estimate image of the area of interest, a further virtual low-energy imaging data can be calculated. The further virtual low-energy data can be compared to the received low-energy imaging data, thereby obtaining a further comparison result. This step can be iteratively repeated to obtain further optimized estimate images until the comparison result fulfills a termination criterion. The last obtained estimated image can be provided as the low-energy image. The comparison result can be based, for example, on a loss function that is applied to a difference or a weighted difference of the calculated virtual low-energy imaging data and the received low-energy imaging data. The termination criterion can be reached, for example, if the deviation of the calculated virtual low-energy imaging data from the received low-energy imaging data is smaller than a predefined threshold.

In another aspect the area of interest comprises a tissue boundary, wherein the initial estimate image comprises an edge representing the tissue boundary, wherein the comparing the calculated virtual low-energy imaging data to the received low-energy imaging data comprises a regularization for preserving the edge in the refined estimate image of the area of interest. Thus smoothing of edges corresponding to tissue boundaries that are already apparent from the computed tomography image can be avoided.

A trained machine learning algorithm, in particular a deep learning algorithm, for example a neural network, can be used for reconstructing the low-energy image or for performing one or more actions within the reconstructing. The trained machine learning algorithm can be applied, for example, to the low-energy imaging data and the computed tomography image. The machine learning algorithm can be trained, for example, based on training data comprising low-energy imaging data and corresponding computed tomography images as an input and corresponding low-energy images as an output. The corresponding low-energy images can be determined, for example based on the low-energy imaging data and the computed tomography images, in particular using an iterative reconstruction according to one or more of the aspects disclosed herein.

In another aspect the method further comprises receiving tissue characteristics atlas data. In a further aspect, the tissue characteristics map of the area of interest is generated based on the tissue characteristics atlas data and the computed tomography image. The tissue characteristics atlas data can comprise anatomy and/or tissue properties. The tissue characteristics atlas data can, for example, be registered to the computed tomography image. Thus, tissue characteristics can be assigned to the voxels of the computed tomography image, thereby obtaining a tissue characteristics map.

In another aspect the tissue characteristics map of the area of interest is generated by applying a trained machine learning algorithm to the computed tomography image. For example, a neural network can be used to classify the voxels with regards to their scattering and/or absorption characteristics.

In another aspect the tissue characteristics map of the area of interest comprises at least one of scattering intensity map, absorption intensity map, refractivity map, and electrical impedance map, or a combination thereof. The refractivity of the tissue, for example, influences the refraction of the low-energy photons, in particular optical photons.

In another aspect the method further comprises registering the low-energy imaging data and the computed tomography image relative to each other. The low-energy image and/or other image information generated based on the low-energy imaging data can be superimposed to the computed tomography image, for example, by using color coding, thereby obtaining an overlay image.

For example, the position of markers, the position of which in a coordinate system of the low-energy imaging data is known, can be determined in the computed tomography image. Such markers can be, for example, sources and/or detectors and/or other components of a low-energy photon source-detector arrangement and/or markers that are external to the low-energy photon source-detector arrangement. The markers can be, for example, radiopaque markers. The low-energy imaging data and the computed tomography image can be registered relative to each other, for example, based on the positions of the markers in the coordinate system of the low-energy imaging data and in the computed tomography image. In order to reduce possible artifacts the markers can be detected and removed within the image space.

In another aspect, the low-energy imaging data and the computed tomography image can be registered based on known positions of sources and/or detectors of a low-energy photon source-detector arrangement relative to a patient transfer board and the position of the patient transfer board in the computed tomography image. An algorithm can be applied for superimposing low-energy imaging data obtained with a detector of the low-energy photon source-detector arrangement to the computed tomography image based on the position of the detector of the low-energy photon source-detector arrangement relative to the patient transfer board.

In another aspect the low-energy imaging data comprises at least one of near-infrared imaging data, microwave imaging data, and electrical impedance tomography data or a combination thereof.

In another aspect the low-energy photons have photon energies of 10 electronvolt per photon or less, in particular 1 electronvolt per photon or less, for example 1 millielectronvolt per photon or less, for example 1 nanoelectronvolt per photon or less.

In one further aspect the invention relates to an imaging data processing unit, comprising
- a low-energy imaging data receiving unit, configured for receiving the low-energy imaging data of an area of interest that is based on an interaction of the area of interest with low-energy photons,
- a computed tomography image receiving unit, configured for receiving a computed tomography image of the area of interest,
- a tissue characteristics map generating unit, configured for generating, based on the computed tomography image, a tissue characteristics map of the area of interest regarding the interaction with the low-energy photons, and
- a reconstructing unit, configured for reconstructing, based on the low-energy imaging data and the tissue characteristics map, the low-energy image.

In another aspect, the imaging data processing unit is configured to implement the method according to one or more of the disclosed aspects.

In one further aspect the invention relates to a medical imaging system, comprising
- a low-energy imaging modality, comprising a low-energy photon source-detector arrangement, configured for acquiring low-energy imaging data of an area of interest,
- a computed tomography device configured for acquiring a computed tomography image of the area of interest, and
- an imaging data processing unit according to one or more of the disclosed aspects.

In another aspect the low-energy imaging modality comprises at least one of a near-infrared imaging device, a microwave imaging device, and an electrical impedance tomography device or a combination thereof.

The high-spatial-resolution images from the computed tomography device can be used to improve the spatial quality of the low-energy image of the low-energy imaging modality that comprises a high amount of functional information. In particular, the computed tomography image can be acquired by the computed tomography device at the same time or shortly before or after the low-energy imaging data, therefore enabling a highly consistent spatial alignment of the computed tomography image and the low-energy imaging data.

In one further aspect the invention relates to a computer program product comprising program elements which induce an imaging data processing unit to carry out the steps of the method according to one or more of the disclosed aspects, when the program elements are loaded into a memory of the imaging data processing unit. In one further aspect the method for reconstructing a low-energy image is a computer-implemented method.

Any of the units mentioned herein or any interface between the units can be embodied in form of hardware and/or software. In particular, an interface can be embodied in form of at least one of a PCI-Bus, a USB or a Firewire. In particular, a unit can comprise hardware elements and/or software elements, for example a microprocessor, a field programmable gate array (an acronym is "FPGA") or an application specific integrated circuit (an acronym is "ASIC").

The imaging data processing unit can, for example, comprise at least one of a cloud-computing system, a distributed computing system, a computer network, a computer, a tablet computer, a smartphone or the like. The imaging data processing unit can comprise hardware and/or software. The hardware can be, for example, a processor system, a memory system and combinations thereof. The hardware can be configurable by the software and/or be operable by the software. Calculations for performing steps of a method and/or for training an algorithm may be carried out in a processor.

Data, in particular, the low-energy imaging data and/or the computed tomography image, can be received, for example, by receiving a signal that carries the data and/or by reading the data from a computer-readable medium. Data, in particular, the tissue characteristics map and/or the low-energy image, can be provided, for example, by transmitting a signal that carries the data and/or by writing the data into a computer-readable medium and/or by displaying the data on a display.

The computer program product can be, for example, a computer program or comprise another element apart from the computer program. This other element can be hardware, for example a memory device, on which the computer program is stored, a hardware key for using the computer program and the like, and/or software, for example, a documentation or a software key for using the computer program. A computer-readable medium can be embodied as non-permanent main memory (e.g. random access memory) or as permanent mass storage (e.g. hard disk, USB stick, SD card, solid state disk).

A computer-readable medium on which program elements are stored that can be read and executed by an imaging data processing unit in order to perform the steps of the method according to one or more of the disclosed aspects, when the program elements are executed by the imaging data processing unit.

Wherever not already described explicitly, individual embodiments, or their individual aspects and features, can be combined or exchanged with one another without limiting or widening the scope of the described invention, whenever such a combination or exchange is meaningful and in the sense of this invention. Advantages which are described with respect to one embodiment of the present invention are, wherever applicable, also advantageous of other embodiments of the present invention.

Any of the algorithms mentioned herein can be based on one or more of the following architectures: convolutional neural networks, deep belief networks, deep residual learning, deep reinforcement learning, recurrent neural networks, Siamese networks, generative adversarial networks or auto-encoders. In particular, the trained machine learning algorithm for providing an optimized energy bin parameter set for photon-counting spectral computed tomography can be embodied as a deep learning algorithm and/or as a convolutional neural network.

Reference is made to the fact that the described methods and the described units are merely preferred example embodiments of the invention and that the invention can be varied by a person skilled in the art, without departing from the scope of the invention as it is specified by the claims.

The invention will be illustrated below with reference to the accompanying figures using example embodiments. The illustration in the figures is schematic and highly simplified and not necessarily to scale.
Fig. 1 shows a diagram illustrating a method for reconstructing a low-energy image,
Fig. 2 shows an imaging data processing unit,
Fig. 3 shows a diagram illustrating a method for reconstructing a low-energy image comprising an iterative reconstruction,
Fig. 4 shows a medical imaging system.

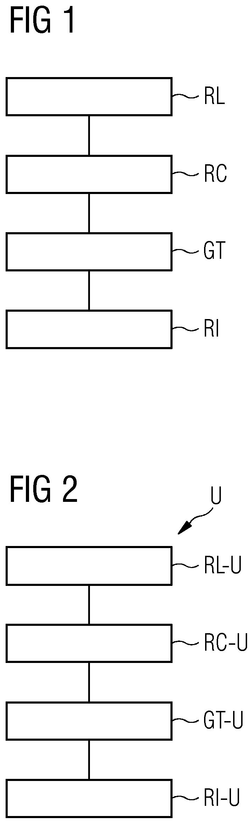

Fig. 1 shows a method for reconstructing a low-energy image,
- Receiving RL the low-energy imaging data of an area of interest 13 that is based on an interaction of the area of interest 13 with low-energy photons,
- Receiving RC a computed tomography image of the area of interest 13,
- Generating GT, based on the computed tomography image, a tissue characteristics map of the area of interest 13 regarding the interaction with the low-energy photons, and
- Reconstructing RI, based on the low-energy imaging data and the tissue characteristics map, the low-energy image.

Fig. 2 shows an imaging data processing unit U, comprising:
- a low-energy imaging data receiving unit RL-U, configured for receiving RL the low-energy imaging data of an area of interest 13 that is based on an interaction of the area of interest 13 with low-energy photons,
- a computed tomography image receiving unit RC-U, configured for receiving RC a computed tomography image of the area of interest 13,
- a tissue characteristics map generating unit GT-U, configured for Generating GT, based on the computed tomography image, a tissue characteristics map of the area of interest 13 regarding the interaction with the low-energy photons, and
- a reconstructing unit RI-U, configured for reconstructing RI, based on the low-energy imaging data and the tissue characteristics map, the low-energy image.

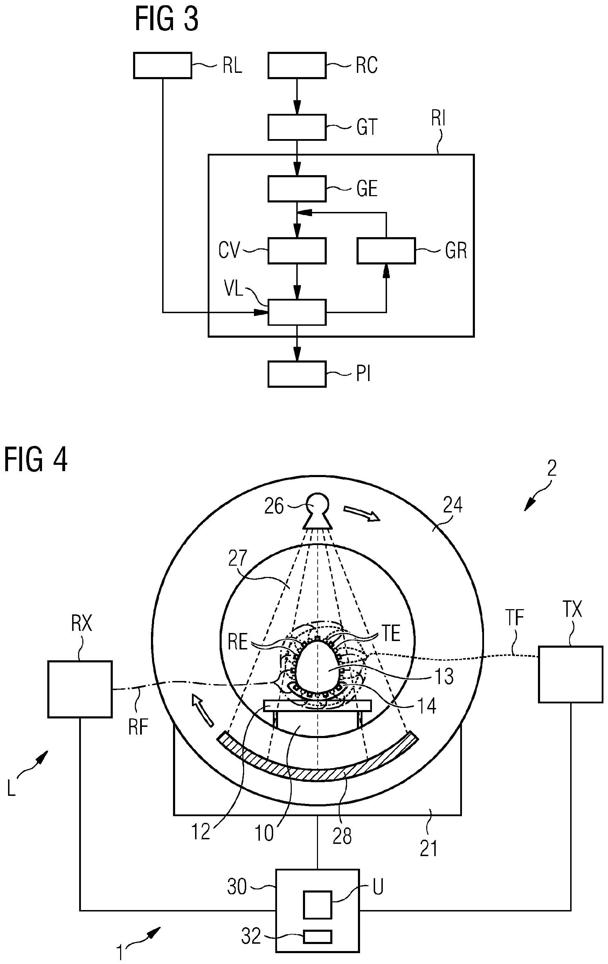

Fig. 3 shows a method for reconstructing a low-energy image, wherein the reconstructing RI the image based on the low-energy imaging data and the tissue characteristics map comprises:
- Generating GE, based on the tissue characteristics map, an initial estimate image of the area of interest 13,
- Calculating CV virtual low-energy imaging data based on the initial estimate image,
- Comparing VL the calculated virtual low-energy imaging data to the received low-energy imaging data, thereby obtaining a comparison result,
- Generating GR, based on the comparison result, a refined estimate image of the area of interest 13.

The actions of CV, VL and GR are repeated iteratively to obtain further estimate images and further comparison results until a termination criterion is fulfilled. The method further comprises providing PI the low-energy image, for example by displaying the low-energy image on a screen and/or by writing the low-energy image into a computer-readable memory.

Fig. 4 shows a medical imaging system, comprising
- a low-energy imaging modality L, comprising a low-energy photon source-detector arrangement TE, RE, configured for acquiring low-energy imaging data of an area of interest 13,
- a computed tomography device 2 configured for acquiring a computed tomography image of the area of interest 13,
- the imaging data processing unit U.

The computed tomography device 2 comprises a support frame 21, a rotating frame 24, an x-ray source 26, an x-ray detector 28, and a patient handling system 10 comprising a patient transfer board 12.

The low-energy photon sources TE, for example, implemented as emitters, and detectors RE are installed within the bore of the computed tomography device 2. The sources TE and detectors RE are distributed around the patient head, which comprises the area of interest 13, in a circumferential or partially circumferential pattern. This configuration is advantageous because the photons are scattered into all directions.

To minimize the absorption of x-ray photons by the low-energy modality, the low-energy modality is constructed in a way to minimize radiation density and artefacts in the computed tomography image. Especially it is beneficial to transport the signal outside the irradiated field and do further signal processing (e.g. amplification) there, which minimizes the placement of electronics in the field of view of the computed tomography device 2.

This can be achieved, for example, by guiding optical signals of a near-infrared imaging device through glass fibers TF from a sending device TX outside the field of irradiation 27 or through glass fibers RF to a receiving device RX outside the field of irradiation 27. Another possible implementation would be to have receivers RE and sender antennas TE, for example, for microwaves that have a minimal radiation density and a geometry that minimizes artefacts in the computed tomography image, in particular, by avoiding sharp edges. At least some of the sources TE and at least some of the detectors RE are placed in a cushion 14 above the head holder. This can be inflated in order to guarantee seamless contact to the head of the patient. Other types of holders can be applied in a similar way to guarantee contact to other parts of the body.

The medical imaging device 1 further comprises a computer 30 to control the computed tomography device 2 and the low-energy imaging device L. The computer 30 comprises the imaging data processing unit U and the computer-readable medium 32.

## Claims

1. Method for reconstructing a low-energy image, comprising:
- Receiving (RL) the low-energy imaging data of an area of interest (13) that is based on an interaction of the area of interest (13) with low-energy photons,
- Receiving (RC) a computed tomography image of the area of interest (13),
- Generating (GT), based on the computed tomography image, a tissue characteristics map of the area of interest (13) regarding the interaction with the low-energy photons, and
- Reconstructing (RI), based on the low-energy imaging data and the tissue characteristics map, the low-energy image.

2. Method according to claim 1, wherein the reconstructing (RI) the image based on the low-energy imaging data and the tissue characteristics map comprises:
- Generating (GE), based on the tissue characteristics map, an initial estimate image of the area of interest (13),
- Calculating (CV) virtual low-energy imaging data based on the initial estimate image,
- Comparing (VL) the calculated virtual low-energy imaging data to the received low-energy imaging data, thereby obtaining a comparison result,
- Generating (GR), based on the comparison result, a refined estimate image of the area of interest (13).

3. Method according to claim 2,
- wherein the area of interest (13) comprises a tissue boundary,
- wherein the initial estimate image comprises an edge representing the tissue boundary,
- wherein the comparing the calculated virtual low-energy imaging data to the received low-energy imaging data comprises a regularization for preserving the edge in the refined estimate image of the area of interest (13).

4. Method according to one of the claims 1 to 3, further comprising:
- Receiving tissue characteristics atlas data,
- wherein the tissue characteristics map of the area of interest (13) is generated based on the tissue characteristics atlas data and the computed tomography image.

5. Method according to one of the claims 1 to 4, wherein the tissue characteristics map of the area of interest (13) is generated by applying a trained machine learning algorithm to the computed tomography image.

6. Method according to one of the claims 1 to 5, wherein the tissue characteristics map of the area of interest (13) comprises at least one of scattering intensity map, absorption intensity map, refractivity map, and electrical impedance map, or a combination thereof.

7. Method according to one of the claims 1 to 6, further comprising:
- Registering the low-energy imaging data and the computed tomography image relative to each other.

8. Method according to one of the claims 1 to 7, wherein the low-energy imaging data comprises at least one of near-infrared imaging data, microwave imaging data, and electrical impedance tomography data or a combination thereof.

9. Method according to one of the claims 1 to 8,
- wherein the low-energy photons have photon energies of 10 electronvolt per photon or less.

10. Imaging data processing unit (U), comprising:
- a low-energy imaging data receiving unit (RL-U), configured for receiving (RL) the low-energy imaging data of an area of interest (13) that is based on an interaction of the area of interest (13) with low-energy photons,
- a computed tomography image receiving unit (RC-U), configured for receiving (RC) a computed tomography image of the area of interest (13),
- a tissue characteristics map generating unit (GT-U), configured for Generating (GT), based on the computed tomography image, a tissue characteristics map of the area of interest (13) regarding the interaction with the low-energy photons, and
- a reconstructing unit (RI-U), configured for reconstructing (RI), based on the low-energy imaging data and the tissue characteristics map, the low-energy image.

11. Imaging data processing unit (U) according to claim 10, configured to implement the method of one of the claims 1 to 9.

12. Medical imaging system (1), comprising
- a low-energy imaging modality (L), comprising a low-energy photon source-detector arrangement (TE, RE), configured for acquiring low-energy imaging data of an area of interest (13),
- a computed tomography device (2) configured for acquiring a computed tomography image of the area of interest (13),
- an imaging data processing unit (U) according to claim 10 or 11.

13. Medical imaging system (1) of claim 12, wherein the low-energy imaging modality (L) comprises at least one of a near-infrared imaging device, a microwave imaging device, and an electrical impedance tomography device or a combination thereof.

14. Computer program product comprising program elements which induce an imaging data processing unit (U) to carry out the steps of the method according to one of the claims 1 to 9, when the program elements are loaded into a memory of the imaging data processing unit (U).

15. A computer-readable medium (32) on which program elements are stored that can be read and executed by an imaging data processing unit (U) in order to perform the steps of the method according to one of the claims 1 to 9, when the program elements are executed by the imaging data processing unit (U).
